# EUROPÄISCHE PATENTSCHRIFT

(11) **EP 3 793 488 B1**
(45) Veröffentlichungstag und Bekanntmachung des Hinweises auf die Patenterteilung: **07.06.2023**
(21) Anmeldenummer: 19722896.8
(22) Anmeldetag: 08.05.2019
(51) Int. Cl.: A61F 2/66

(54) **KUNSTFUSS**
ARTIFICIAL FOOT
PIED ARTIFICIEL

(30) Priorität: 14.05.2018 DE 202018102690 U
(43) Veröffentlichungstag der Anmeldung: 24.03.2021
(73) Patentinhaber: Mecuris GmbH, 80337 München (DE)
(72) Erfinder: BREUNINGER, Jannis, 72663 Großbettlingen (DE); GLAS, Franziska, 80686 München (DE); ROCHLITZ, Bence, 80686 München (DE); TAUBMANN, Carolin, 91284 Neuhaus (DE); RIETH, Clemens, 71032 Böblingen (DE)
(74) Vertreter: Meissner Bolte Partnerschaft mbB
(86) Internationale Anmeldenummer: PCT/EP2019/061839
(87) Internationale Veröffentlichungsnummer: WO 2019/219483

(56) Entgegenhaltungen:
- WO-A1-01/06965
- DE-A1-102015 101 746
- GB-A- 306 313
- US-A1- 2010 042 228

## Beschreibung

Die Erfindung bezieht sich auf einen Kunstfuß für den Einsatz in einer Bein- oder Fußprothese.

Ein Kunstfuß ist aus der DE 20 2015 007 994 bekannt. Kunstfüße bzw. Prothesenfüße sind außerordentlich hohen Belastungen ausgesetzt. Bisherige Prothesenfüße werden daher im Spritzgussverfahren oder Laminierverfahren aus Kunststoffen, faserverstärkten Kunststoffen und/oder aus Metalllegierungen hergestellt. Aus der DE 10 2014 006 727 B3 sind Prothesenfüße bekannt, die mittels additiver Fertigungsverfahren, welche auch unter dem Begriff 3D-drucken bekannt sind, hergestellt werden.

Für die Erfüllung der funktionellen Anforderungen des Kunstfußes sind mehrere Aspekte wichtig. Die Dämpfungseigenschaften, welche sich am deutlichsten beim Fersenauftritt zeigen, sind wichtig für den Komfort beim Gehen. Die Geometrie der Sohle ist wichtig für ein harmonisches Abrollverhalten während des Gehens. Ebenso ist eine, zumeist durch passive Federelemente erreichte, Energierückgabe beim Ablösen des Vorfußes vom Boden erwünscht. Dies sorgt für ein energieeffizientes Gehen des Prothesenträgers.

Generell ist eine individuelle, Patientenspezifische Auslegung der gesamten Prothese wichtig, um die funktionellen Elemente auf die Bedürfnisse des Patienten anzupassen. Herkömmliche Prothesenfüße lassen hier jedoch nur bedingt individuelle Veränderungen zu. Zumeist wird nicht der Prothesenfuß an sich verändert, sondern der Orthopädietechniker wählt aus der Vielzahl verschiedener Prothesenfüße einen Fuß aus, der von den Eigenschaften am ehesten den gewünschten Eigenschaften entspricht. Zudem sind Prothesenfüße zumeist in verschiedenen Größen verfügbar. Einige können noch durch beispielsweise den Austausch oder die Einstellung von Dämpfungselementen angepasst werden.

So offenbart die Druckschrift DE 10 2015 101 746 A1 als nächstliegender Stand der Technik einen Prothesenfuß mit einem Federelement, das mit dem Sohlenbereich fest verschraubt ist.

Die zum jetzigen Zeitpunkt noch in der Entwicklung befindlichen additiv gefertigten Prothesenfüße bieten durch die werkzeuglose Fertigung die Möglichkeit jeden einzelnen Kunstfuß individuell anzupassen. Somit kann hier nicht nur die Fußgrö-ße angepasst werden, sondern im besonders hohen Maße auch die Dämpfungseigenschaften sowie das Abrollverhalten. Jedoch bieten die für diese Verfahren angebotenen Materialien zum heutigen Zeitpunkt nicht die Federeigenschaften und somit die Energierückgabe die beispielsweise mit herkömmlich gefertigten, faserverstärkten Kunstsoffen erreichbar sind.

Ausgehend von diesem Stand der Technik ist es Aufgabe der vorliegenden Erfindung, einen verbesserten Kunstfuß, insbesondere eine verbesserte Fußprothese, anzugeben. Weiterhin ist es Aufgabe, den Kunstfuß derart auszugestalten, dass in einfacher und kostengünstiger Weise eine höhere Individualisierung ermöglicht wird. Die Herstellungskosten sollen möglichst gering und der Komfort des Nutzers möglichst hoch sein.

Die Aufgabe wird durch den Gegenstand gemäß Anspruch 1 gelöst.

Insbesondere wird die Aufgabe durch einen Kunstfuß gelöst, der einen Grundkörper mit einem vorderen Sohlenbereich und einen Fersenkörper mit einem hinteren Sohlenbereich umfasst. Der Kunstfuß umfasst kann einen Einschub zur Aufnahme mindestens eines einschiebbaren und/oder einsteckbaren Federelements. Bei dem Federelement kann es sich um Stabfedern oder Blattfedern handeln. Erfindungsgemäß greift das Federelement in einem angeordneten Zustand derart in einen Grundkörper und den Fersenkörper ein, dass der Fersenkörper gegenüber dem Grundkörper federnd gelagert ist.

Ein Kern der Erfindung besteht also darin, einen Kunstfuß zu schaffen, bei dem wesentliche strukturelle Elemente einfach, insbesondere in einem additiven Verfahren, individuell hergestellt werden, wobei das Verhalten des Kunstfußes beim Gehen oder Laufen durch individuell auswählbare Federelemente eingestellt werden kann. Die Auswahl der Federelemente kann basierend auf Gewichtsangaben und/oder Anforderungsprofilen ausgewählt werden.

Der vordere und/oder hintere Sohlenbereich muss nicht zwangsläufig aus Gummi hergestellt sein. Erfindungsgemäß bezeichnet er lediglich einen Bereich, der mit der biologischen Fußsohle korreliert. Der erfindungsgemäße Kunstfuß kann derart ausgestaltet sein, dass er offen - also ohne die Verwendung einer Kosmetik - getragen wird. Es ist aber auch möglich, den Gedanken der vorliegenden Erfindung auf einen Kunstfuß anzuwenden, der zusammen mit einer Kosmetik verwendet wird.

Erfindungsgemäß kann der oben verwendete Begriff der federnden Lagerung derart definiert sein, dass hier für das Bewegen des Fersenkörpers insbesondere in horizontale Richtung eine Federkraft überwunden wird, die von dem besagten Federelement aufgebracht wird.

In einer Ausführungsform ist das Federelement mit dem Grundkörper verbunden. Es ist möglich, dass das Federelement "schwimmend" in einer Öffnung oder Aussparung des Grundkörpers und/oder des Fersenkörpers liegt. Bevorzugt gibt es eine Befestigung, wobei die Befestigung über eine Klebeverbindung und/oder eine Schraubverbindung und/oder eine Rastverbindung und/oder eine Verbindung über eine Presspassung hergestellt werden kann. Reversible Befestigungen sind zu bevorzugen. Dabei kann eine feste Verbindung in mindestens einem Bereich und/oder in zwei Bereichen, z.B. vorn und mittig oder hinten und mittig vorgesehen sein. In einer Ausführungsform kann vorgsehen sein, dass das Federlement in zwei Bereichen "schwimmend" angeordnet ist, z.B. vorne und mittig oder hinten und mittig oder vorne und hinten. In einer Ausführungsform kann das Federelement (nur) in einem vorderen Bereich und/oder in einem hinteren Bereich mit dem Grundkörper über eine Befestigung verbunden sein. In einer Ausführungsform ist es auch möglich, dass das Federelement in einem vorderen und einem hinteren Bereich mit dem Grundkörper verbunden ist. Somit entsteht eine Anordnung, bei das Federelement fest und nicht "schwimmend" befestigt ist.

In einer Ausführungsform liegt das Federelement mittig an dem Grundkörper an, so dass sich an diesem Anlagepunkt eine Rotationsachse oder ein Rotationsbereich ergibt, um die sich das Federelement drehen kann. Diese mittige Anlage kann dazu führen, dass beim Aufkommen des Kunstfußes ein Eindrücken der Ferse - Wegfedern - den vorderen und/oder mittleren Bereich des Kunstfußes derart krümmt, dass der mittlerer Bereich des Kunstfußes relativ früh mit dem Boden in Kontakt kommt. Diese Art der Verformung des Kunststoffs führt zu einer besonders komfortablen Nutzbarkeit des Kunstfußes.

In einer Ausführungsform befindet sich im mittigen Bereich auch die Verbindung zwischen dem Federelement und dem Grundkörper.

Erfindungsgemäß kann mittig bzw. mittiger Bereich derart definiert sein, dass es sich hier um einen Bereich nahe des Mittelpunkts des Federelements handelt. Beispielsweise kann dieser Bereich einen Bereich umfassen, der maximal 20 %, insbesondere maximal 10 % der Gesamtlänge von dem Mittelpunkt des Federelements entfernt ist.

In einer Ausführungsform kann das Federelement im vorderen Bereich des Grundkörpers und/oder im hinteren Bereich des Fersenkörpers in Längsrichtung des Federelements verschiebbar, insbesondere in einer Öffnung gelagert sein.

Wie bereits erläutert kann es sich bei dem Federelement um ein längliches Federelement, beispielsweise um eine Blattfeder oder um einen Federstab handeln. Theoretisch ist es auch möglich, dass mehrere Blattfedern oder mehrere Federstäbe verwendet werden. Die Feder kann aus einem faserverstärkter Kunststoff hergestellt sein. Es sind jedoch auch andere Materialien wie federnde Metalle, insbesondere Federstahl oder Kunststoffe ohne Fasern denkbar.

Es ist vorteilhaft, wenn sich zumindest Abschnitte des Kunstfußes gegenüber dem Federelement in Längsrichtung des Federelements verschieben können. Durch diese Verschiebbarkeit kann sich der Kunstfuß beim Gehen oder Laufen noch stärker verformen und so einen dynamischen und komfortablen Auftritt ermöglichen.

Diese Verschiebbarkeit wird vorzugsweise dadurch erreicht, dass Endabschnitte des Federelements in Öffnungen gelagert sind. Diese Öffnungen sind vorzugsweise derart ausgestaltet, dass sie in der vertikalen Richtung mit oder ohne Spiel an dem Federelement anliegen. In horizontaler Richtung - bezogen auf die Längsachse des Federelements - ist Platz vorhanden, so dass sich das Federelement innerhalb der Öffnung verschieben kann. Bei der besagten Öffnung kann es sich im Fersenkörper um den bereits genannten Einschub handeln.

Der Grundkörper kann einen Vorderfuß oder Vorfußkörper mit dem vorderen Sohlenbereich und einen Befestigungskörper, insbesondere zur Aufnahme eines Adapters umfassen. Derartige Adapter sind bekannt und teilweise bereits standardisiert, um ein schnelles und effektives Anbringen des Kunstfußes über geeignete Mittel am Körper des Nutzers zu ermöglichen.

Der Vorderfußkörper kann flexibel insbesondere über einen Steg, mit dem Befestigungskörper verbunden sein. Insofern kann sich der Vorderfußkörper gegenüber dem Adapter bewegen und Kräfte aufnehmen und abgeben. Die flexible Verbindung in Form eines Steges wird also durch die Struktur des Kunstfußes unterstützt. Der Steg nimmt also die Funktion eines Federelements wahr.

In einer Ausführungsform ist der Fersenkörper über einen Fersensteg mit dem Grundkörper verbunden. Vorzugsweise erstreckt sich dieser Steg im Wesentlichen (max. ± 30°) in horizontaler Richtung, so dass das Fersenelement in vertikale Richtung einfedern kann. Der Steg kann zumindest abschnittsweise parallel zu dem Federelement verlaufen. In einer Ausführungsform umgreift der Steg das Federelement zumindest entlang eines Abschnitts. Beispielsweise kann der Steg einen Querschnitt eines (umgedrehten) U-Profils oder C- Profils haben. Es ist möglich, dass der Steg ebenfalls die Funktion eines Federelements übernimmt. Letztendlich kann die Flexibilität des Fersenkörpers gegenüber dem Grundkörper durch eine geeignete Dimensionierung des Stegs und des Federelements eingestellt werden. In einer Ausführungsform weisen die Materialien die zur Herstellung des Stegs verwendet werden und die das Federelement bilden, unterschiedliche Federeigenschaften auf. Durch eine gezielte Wahl der Dimensionen können hochgradig individuelle Abrollkurven eingestellt werden.

Der vordere Sohlenbereich kann zumindest abschnittsweise vorzugsweise über mindestens 50% oder 30% der Kontaktfläche, eine konvexe Form haben. Die Kontaktfläche kann hierbei so definiert werden, dass es die Fläche ist, die bei einer üblichen Geh- oder Laufbewegung auf einem ebenen Untergrund mit dem Boden in Kontakt kommt.

Der Grundkörper kann ein Anschlagelement umfassen, das sich nach unten hin erstreckt. Dieses Anschlagelement kann an dem Federelement mittelbar oder unmittelbar anliegen. Vorzugsweise ist das Anschlagelement derart ausgestaltet, dass der Bereich, an dem das Anschlagelement mittelbar oder unmittelbar an dem Federelement anliegt, im vorderen Bereich, insbesondere im vorderen Drittel des Federelements liegt. Das Anschlagelement leitet Kräfte, die auf das Federelement wirken, in den Adapter ein. Beim Auftreten kann die Feder um den Bereich rotieren, an dem das Anschlagelement an dieser anliegt.

Das Anschlagelement kann sich im Wesentlichen von dem Adapterelement nach vorne unten erstrecken. In einer Ausführungsform ist eine Längsachse des Anschlagelements um ca. 30° gegenüber einer Adapterebene geneigt.

Das Federelement kann im Wesentlichen parallel zur Adapterebene und/oder in einer nach vorne hin ansteigenden Ebene angeordnet sein. Erfindungsgemäß kann bei einer ansteigenden Anordnung ein Winkel von 1-5°, vorzugsweise 4,4°, insbesondere von 1-3°, besonders bevorzugt 1,8°, eingenommen werden. Gegenüber der Bodenebene besteht vorzugsweise ein Winkel des Federelements von 1-10°. Das Federelement ist im hinteren Bereich also relativ bodennah, während es im vorderen Bereich deutlich stärker beabstandet ist. In einer Ausführungsform ist der Kunstfuß derart ausgebildet, dass das Federelement insgesamt relativ bodennah, d.h. beispielsweise maximal 3 cm vom Boden entfernt angeordnet ist.

In einer Ausführungsform kann der Winkel zwischen Federelement und Bodenebene von der Höhe des Fersenkörpers abhängen. Bei einer Höhe des Fersenkörpers von 12mm kann z.B. sich z.B. ein Winkel von 4,4° ergeben. Somit ist es möglich, den Kunstfuß derart auf einen Schuh abzustimmen, in dem der Kunstfuß getragen werden kann, das der Winkel zwischen einer Bodenebene und dem Federelement zumindest im Wesentlichen ca. 0° (±2°) beträgt, wenn der Kunstfuß in dem Schuh angeordnet ist.

Der Grundkörper kann einen hinteren Anschlag umfassen, der über einen Spalt von dem Fersenkörper entfernt ist. Der Spalt ermöglicht also ein Schwingen bzw. Federn des Fersenkörpers gegenüber dem Grundkörper, insbesondere gegenüber dem Anschlag. Bei einer Maximalbelastung liegt der Fersenkörper jedoch an dem Anschlag an, so dass eine Beschädigung des Kunstfußes vermieden werden kann. Der Anschlag ist hierfür vorzugsweise relativ solide ausgebildet.

In einer Ausführungsform ist der Anschlag derart ausgebildet, dass der Fersenkörper bei Maximalbelastung über eine größere Fläche, mindestens 1 cm², vorzugsweise mindestens 2 cm² an dem Anschlag anliegt.

Der Kunstfuß kann in einem additiven Verfahren hergestellt sein. Vorzugsweise wird der Kunstfuß mittels 3D-Druck hergestellt. Hierbei kann ein Thermoplast verwendet werden.

Der Grundkörper und/oder Fersenkörper und/oder Vorderfußkörper und/oder Befestigungskörper und/oder mindestens einer der Stege und/oder das Anschlagelement und/oder weitere Elemente können integral also als ein Element hergestellt sein.

Die eingangs genannte Aufgabe wird auch durch ein Verfahren zur Herstellung eines Kunstfußes mit den bereits beschriebenen Merkmalen erreicht.

Weitere vorteilhafte Ausführungsformen ergeben sich anhand der Unteransprüche.

Nachfolgend wird die Erfindung mittels mehrerer Ausführungsbeispiele beschrieben, die anhand von Abbildungen näher erläutert werden. Hierbei zeigen:
- Fig. 1: einen ersten Prothesenfuß, wobei der Grundkörper und der Fersenkörper über einen Fersensteg verbunden sind;
- Fig. 2: einen zweiten Prothesenfuß, bei dem Grundkörper und Fersenkörper lediglich über eine Blattfeder verbunden sind;
- Fig. 3: einen dritten Prothesenfuß;
- Fig. 4: einen Längsschnitt durch den ersten Prothesenfuß aus Fig. 1;
- Fig. 5: eine erste perspektivische Ansicht des Prothesenfußes aus Fig. 1 (von oben);
- Fig. 6: eine zweite perspektivische Ansicht des Prothesenfußes aus Fig. 1 (von unten);
- Fig. 7: eine exemplarische Seitenansicht einer Blattfeder;
- Fig. 8: einen vierten Prothesenfuß.

In der nachfolgenden Beschreibung werden für gleiche und gleichwirkende Teile dieselben Bezugsziffern verwendet.

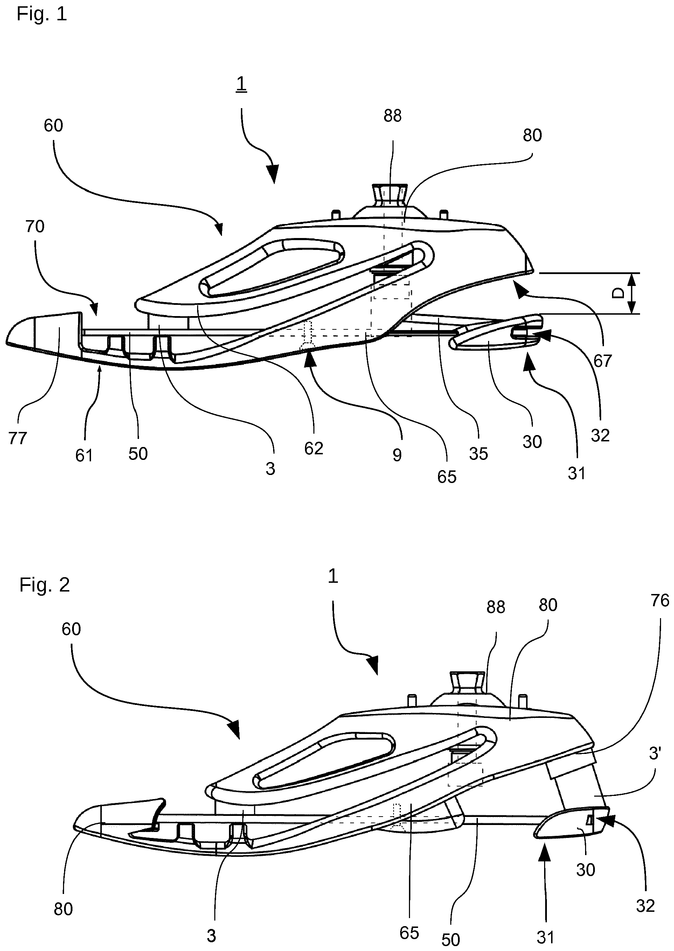

Fig. 1 zeigt eine Seitenansicht eines erfindungsgemäßen Prothesenfußes 1. Dieser setzt sich im Wesentlichen aus zwei Elementen, nämlich einem Fersenkörper 30 und einem Grundkörper 60 zusammen. Fersenkörper 30 und Grundkörper 60 sind im in Fig. 1 gezeigten Ausführungsbeispiel über einen Fersensteg 35 miteinander verbunden. Der Fersensteg erstreckt sich im Wesentlichen horizontal. Grundkörper 60, Fersensteg 35 und Fersenkörper 30 bilden eine integrale Einheit, die in einem additiven Verfahren, beispielsweise durch 3D-Druck hergestellt sind.

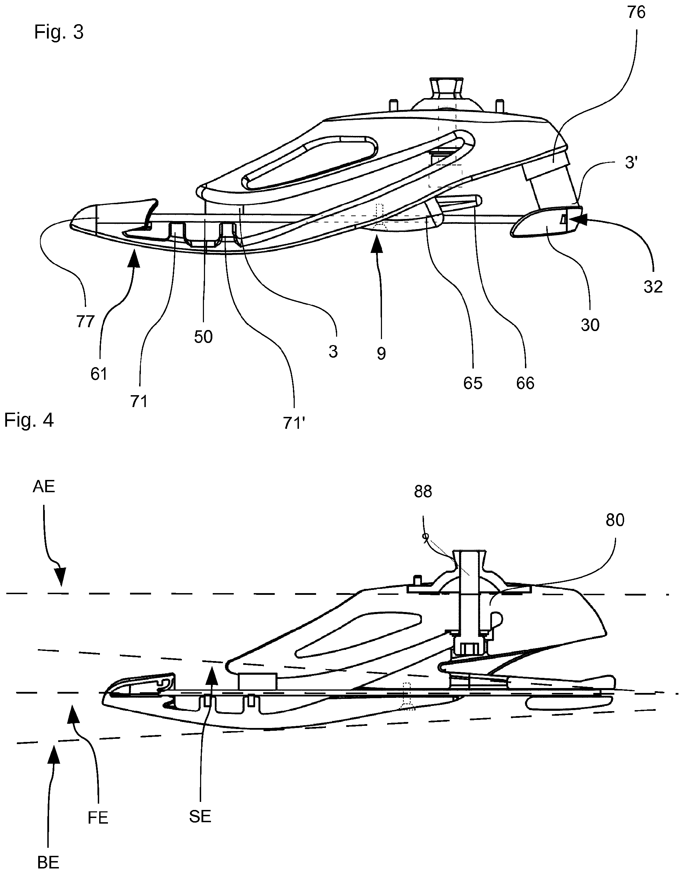

Der Fersenkörper 30 weist einen Einschub 32 mit entsprechender Einschuböffnung auf, in die eine Blattfeder 50 eingeführt ist. Die Blattfeder 50 erstreckt sich in Längsrichtung des Prothesenfußes 1, im Wesentlichen horizontal zu einer Bodenebene BE (Fig. 4). Die Blattfeder 50 hat eine Länge die im Wesentlichen der Gesamtlänge des Prothesenfußes entspricht. Sie ist lediglich um 15 % kürzer als die Gesamtlänge des Prothesenfußes 1. Die Blattfeder 50 verstärkt den Fersensteg 35 und sorgt für eine federnde Lagerung des Fersenkörpers 30.

Der hintere Teil des Grundkörpers 60 mit einem hinteren Anschlag 67 ist deutlich von dem Fersenkörper beabstandet. Hier erstreckt sich ein Spalt, der eine Spalthöhe D von mindestens 1 cm hat. Im gezeigten Ausführungsbeispiel ist die Spalthöhe D 2 cm. Der Spalt ermöglicht eine freie Bewegung des Fersenkörpers 30 in vertikale Richtung. Bei einer Überbelastung schlägt der Fersenkörper 30 an dem hinteren Anschlag 67 an und verhindert so, dass der Fersensteg 35 und/oder die Blattfeder 50 übermäßigen Belastungen ausgesetzt sind, was möglicherweise zu einem Bruch führen könnte.

Der Grundkörper 60 lässt sich funktionell in weitere Elemente unterteilen. Hierbei handelt es sich um einen Vorderfußkörper 70, einen Befestigungskörper 80, einen Steg 65 sowie ein Anschlagelement 62. All diese Elemente sind integral miteinander verbunden. Der Steg 65 sorgt für eine elastische Verbindung zwischen dem Befestigungskörper 80 und dem Vorderfußkörper 70. Der Befestigungskörper 80 nimmt an seiner Oberseite einen Adapter 88 auf. Der Steg 65 erstreckt sich im Wesentlichen von oben rechts nach links unten und geht fließend in den Vorderfußkörper 70 über. Der Steg 65 hat eine durchschnittliche Stärke von ca. 2 cm. Erfindungsgemäß kann der Steg 65 eine durchschnitte Stärke von 1 bis 5 cm, insbesondre von 1 bis 3 cm haben

An der Unterseite des Vorderfußkörpers 70 befindet sich ein vorderer Sohlenbereich 61. Ein korrespondierender hinterer Sohlenbereich 31 ist an der Unterseite des Fersenkörpers 30 angeordnet. In dem in Fig. 1 gezeigten Ausführungsbeispiel ist am hinteren Sohlenbereich 31 und am vorderen Sohlenbereich 61 eine Gummierung vorgesehen, die für einen guten Bodenkontakt sorgt.

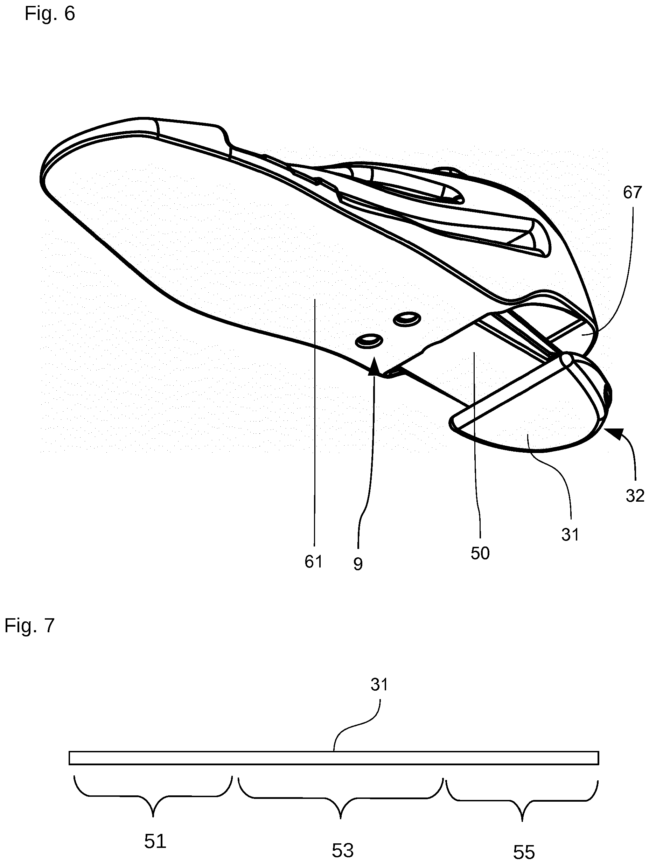

Der Vorderfußkörper 70 verfügt weiterhin über ein Kappe 77 mit einer Aufnahme (vgl. Fig. 4), in die die Blattfeder 50 hineinragt. Mittig des Prothesenfußes 1 auf der Höhe des Stegs 65 ist die Blattfeder 50 mit dem Grundkörper 60 über zwei Schrauben 9 (vgl. auch Fig. 6) verschraubt. Die Schrauben 9 sichern die Blattfeder 50 gegen einen Verschub in Längsrichtung ab. An ihren Enden ist die Blattfeder 50 nicht mit dem Prothesenfuß verbunden und ragt jeweils lose in die dort vorgesehenen Öffnungen hinein, so dass sich beispielsweise die Kappe 77 beim Gehen gegenüber der Blattfeder 50 in Längsrichtung verschieben kann. Die Blattfeder 50 ist über zwei Querstege 71, 71' (vgl. Fig. 3) gegenüber dem Vorderfußkörper 70 abgestützt. Der erste, vordere Quersteg 71 und der zweite, hintere Quersteg 71' sind integrale Bestandteile des Vorderfußkörpers 70. Sie können ihrerseits als Federelemente ausgebildet sein oder eine federnde Wirkung haben.

Das Anschlagelement 62 bildet den oberen Bereich des Grundkörpers 60 und verläuft im Wesentlichen parallel zu dem Steg 65. Steg 65 und Anschlagelement 62 sind voneinander beabstandet. Es ergibt sich ein Spalt von ca. 1 cm, der nach hinten oben hin über die Lastlinie und/oder Aufbaulinie des Kunstfußes 1 hinausreicht. Der Spalt verläuft im Wesentlichen (ca. +/-15%) in einem 45-Grad-Winkel zur Längsachse der Blattfeder 50.

Im vorderen unteren Bereich des Anschlagelements 62 ist ein Dämpfer 3 angeordnet, der unmittelbar auf der Oberseite der Blattfeder 50 aufliegt. Auf der Unterseite der Blattfeder 50 setzt an dieser Stelle der zweite Quersteg 71' an. Der zweite Quersteg 71' und der Anschlagpunkt bzw. Anschlagbereich des Anschlagelements 62 befinden sich also in Längsrichtung im Wesentlichen an derselben Position.

Fig. 2 zeigt ein weiteres Ausführungsbeispiel des erfindungsgemäßen Prothesenfußes 1. Hier wurde auf den Fersensteg 35 aus Fig. 1 verzichtet. Stattdessen ist der Fersenkörper 30 als gesondertes Element ausgebildet. Der Fersenkörper 30 und der Grundkörper 60 sind also nicht integral miteinander verbunden. Die Blattfeder 50 ist im Wesentlichen in entsprechender Weise angeordnet, wie dies bereits anhand des Prothesenfußes 1 aus Fig. 1 erläutert wurde. Erfindungsgemäß können zusätzliche Befestigungsmittel im Fersenkörper 30 vorgesehen sein, um ein Verschieben des Fersenkörpers 30 Längsrichtung des Federelements 50 zu verhindern. Auch der Fersenkörper 30 der Fig. 2 hat einen Einschub 32, in dem das Federelement 50 steckt. Auch der Fersenkörper 30 ist derart federnd gelagert, dass eine Bewegung in vertikaler Richtung möglich ist. And er Oberseite des Fersenkörpers 30 setzt ein Dämpfer 3' an, der mit dem Anschlag 76 verbunden ist. In einer Ausführungsform weist der Fersenkörper 30 keine zusätzlichen Befestigungsmittel auf. Die Längsverschiebbarkeit des Fersenkörpers 30 gegenüber dem Grundkörper 60 wird durch den Dämpfer 3 sowie durch den Anschlag 76 begrenzt.

Fig. 3 zeigt einen Prothesenfuß 1, der im Wesentlichen dem Prothesenfuß aus Fig. 2 entspricht.

Ein Unterschied besteht darin, dass der Steg 65 eine Nase 66 aufweist. Die Nase 66 ist oberhalb der Blattfeder 50 angeordnet und ersteckt sich im Wesentlichen in Richtung des Fersenkörpers 30 horizontal zur Bodenebene. Die Nase 66 erstreckt sich im gezeigten Ausführungsbeispiel ca. über 40% der Strecke zwischen Steg 65 und Fersenkörper 30. Wird die Blattfeder 50 in vertikaler Richtung verformt, so wirkt die Nase 66 als Anschlagelement, sodass die Bewegung der Blattfeder 50 zumindest über die Länge der Nase 66 begrenzt wird. Die Blattfeder 50 legt sich bei Belastung flächig an die Nase 66 an.

Fig. 4 zeigt einen Querschnitt durch den Prothesenfuß 1 aus Fig. 1. Mittels dieses Querschnitts lassen sich relevante Ebenen definieren, die zur Angabe der Position der Blattfeder 50 sowie des Fersenstegs 35 notwendig sind. So spannt der Adapter 88 durch seine Befestigungspunkte am Grundkörper 60 eine Adapterebene AE auf, die sich im Wesentlichen horizontal erstreckt. Eine Bodenebene BE verläuft im Wesentlichen parallel (ca. - 20°) zu der Adapterebene AE. Die Federebene FE, in der die Blattfeder 50 liegt, verläuft im Wesentlichen parallel zur Adapterebene AE. Im gezeigten Ausführungsbeispiel steigt die Federebene nach vorne hin um ca. 5° an. Der Fersensteg 35 erstreckt sich entlang einer Fersenstegebene FE. Diese Fersenstegebene FE ist deutlich stärker gegenüber der Adapterebene AE und der Bodenebene BE geneigt als die Federebene FE. Der sich ergebende Winkel liegt zwischen 10 und 15°.

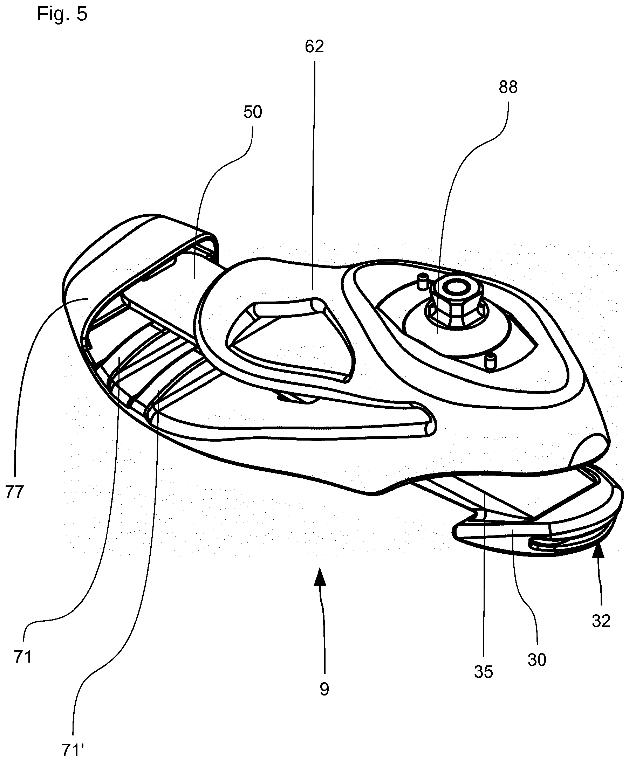

Fig. 5 und 6 zeigen weitere perspektivische Ansichten des Prothesenfußes 1 aus Fig. 1.

Fig. 7 zeigt eine erfindungsgemäße Blattfeder 50, wobei diese in drei Abschnitte, nämlich einen vorderen Bereich 51, einen mittleren Bereich 53 und einen hinteren Bereich 55 unterteilt ist. Der vordere Bereich 51 und der hintere Bereich 55 nehmen im Wesentlichen ein Viertel der Gesamtlänge der Blattfeder 50 ein. Der mittlere Bereich erstreckt sich über ca. zwei Viertel der Gesamtlänge der Blattfeder 50. Die bereits erläuterte Befestigung mittels der Schrauben 9 wird im mittleren Bereich 53 vorgenommen. Die Querstege 71, 71' liegen im Übergangsbereich vom vorderen Bereich 51 zum mittleren Bereich 53 an der Blattfeder 50 an. In diesem Bereich liegt auch das Anschlagelement 62 über dem Dämpfer 3 an der Blattfeder 50 an.

Der hintere Bereich 55 wird größtenteils von dem Einschub 52 des Fersenkörpers 30 aufgenommen.

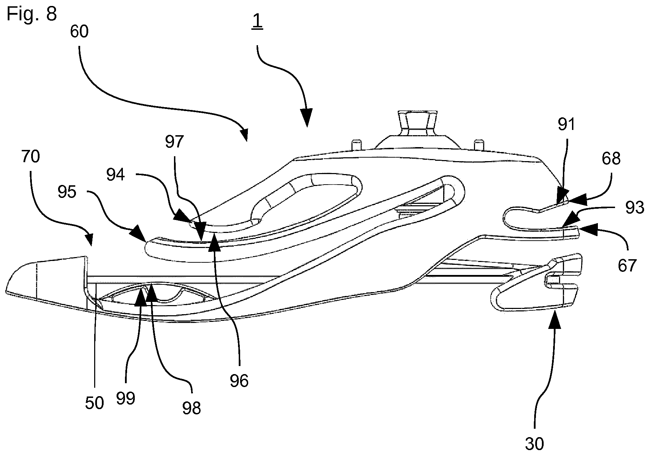

Fig. 8 zeigt eine weitere Ausführungsform eines Prothesenfußes 1, der im Wesentlichen den Prothesenfüßen 1 der Figuren 1 bis 7 entspricht.

Im Unterschied zu den Prothesenfüßen der Figuren 1 bis 3 weist der Prothesenfuß 1 der Figur 8 im hinteren Bereich des Prothesenfußes 1 einen gedämpften Anschlag 67 auf. Dabei weist der Anschlag 67 einen im Wesentlichen flächigen unteren Anschlagsbereich 93 auf, der unter Belastung gegen einen oberen Anschlagsbereich 91 eines hinteren Endanschlags 68 gedrückt wird. Der untere Anschlagsbereich 93 ist korrespondierend zu dem oberen Anschlagsbereich 91 derart ausgebildet, dass der untere Anschlagsbereich 93 unter Last flächig mit dem oberen Anschlagsbereich 91 in Kontakt kommt. Dies gewährleistet eine gleichmäßige Krafteinleitung in den Prothesenfuß 1.

Der untere Anschlagsbereich 93 verläuft im unbelasteten Zustand zumindest im Wesentlichen parallel zu einer Bodenebene. Bei Belastung des Fersenkörpers 30 wird dieser gegen den hinteren Anschlag 67 gedrückt, sodass dieser als eine Feder wirkt und gegen den hinteren Endanschlag 68 gedrückt wird.

Darüber hinaus weist der Prothesenfuß 1 einen Steg 98 auf, über den die Blattfeder 50 gegenüber dem Vorderfußkörper 70 abgestützt ist. Der Steg 98 ist über einen Steghalter 99 gebogen, sodass die Blattfeder 50 auch bei einer Verformung des Vorderfußkörpers 70 immer in Kontakt mit dem Steg 98 bleibt.

Das Anschlagselement 62 des Grundkörpers 60 ist im Ausführungsbeispiel der Figur 8 im vorderen Bereich (Richtung Vorfuß) geteilt ausgeführt, sodass eine weitere Dämpfungswirkung bereitgestellt wird. Das Anschlagselement 62 umfasst in dem Ausführungsbeispiel der Figur 8 einen oberen Anschlagsbereich 94 und einen unteren Dämpfungsbereich 95. Der untere Dämpfungsbereich 95 ist oberhalb der Blattfeder 50 angeordnet, sodass die Blattfeder 50 bei Belastung auf den Vorderfußkörper 70 bei einer Gehbewegung gegen den unteren Dämpfungsbereich 95 gedrückt wird. Der untere Dämpfungsbereich 95 ist flexibel ausgebildet, sodass der untere Dämpfungsbereich 95 bei Belastung gegen den oberen Anschlagsbereich 94 gedrückt wird. Somit werden insgesamt bessere Dämpfungseigenschaften bereitgestellt, was einen höheren Tragekomfort für den Benutzer zur Folge hat.

An dieser Stelle sei darauf hingewiesen, dass alle oben beschriebenen Teile für sich allein gesehen und in jeder Kombination, insbesondere der in den Zeichnungen dargestellten Details als erfindungswesentlich beansprucht werden. So sind beispielsweise zahlreiche Variationen hinsichtlich des Neigungswinkels des Fersenstegs 35 und/oder der Blattfeder 50 denkbar. Ebenso kann die Blattfeder 50 durch eine oder mehrere Blattfedern 50 und/oder Federstege ersetzt werden.

### Bezugszeichenliste:

- 1: Prothesenfuß
- 3, 3': Dämpfer
- 9: Schraube
- 30: Fersenkörper
- 31: hintere Sohlenbereich
- 32: Einschub
- 35: Fersensteg
- 50: Blattfeder
- 51: Vorderer Bereich der Blattfeder
- 53: Mittlerer Bereich der Blattfeder
- 55: Hinterer Bereich der Blattfeder
- 60: Grundkörper
- 61: vordere Sohlenbereich
- 62: Anschlagelement
- 65: Steg
- 66: Nase
- 67: hinterer Anschlag
- 68: hinterer Endanschlag
- 70: Vorderfußkörper
- 71, 71': Quersteg
- 77: Kappe
- 80: Befestigungskörper
- 88: Adapter
- 91: oberer Anschlagsbereich
- 93: unterer Anschlagsbereich
- 94: oberer Anschlagsbereich
- 95: unterer Dämpferabschnitt
- 96: untere Dämpferfläche
- 97: obere Dämpferfläche
- 98: Steg
- 99: Steghalter
- AE: Adapterebene
- FE: Federebene
- SE: Fersenstegeben
- BE: Bodenebene
- D: Spalthöhe

## Patentansprüche

1. Kunstfuß, insbesondere Fußprothese (1), umfassend einen Grundkörper (60) mit einem vorderen Sohlenbereich (61) und einen Fersenkörper (30) mit einem hinteren Sohlenbereich (31),
**dadurch gekennzeichnet, dass**
der Kunstfuß einen Einschub (32) zur Aufnahme mindestens eines einschiebbaren und/oder einsteckbaren Federelements (50), insbesondere mindestens einer Blattfeder umfasst,
und das Federelement (50) in einem angeordneten Zustand derart in den Grundkörper (60) und den Fersenkörper (30) eingreift, dass der Fersenkörper (30) gegenüber dem Grundkörper (60) federnd gelagert ist.

2. Kunstfuß nach Anspruch 1,
**dadurch gekennzeichnet, dass**
das Federelement (50) im angeordneten Zustand mit dem Grundkörper (60), insbesondere mittig, verbunden ist, wobei vorzugsweise eine Klebeverbindung und/oder eine Schraubverbindung und/oder eine Rastverbindung und/oder eine Verbindung über eine Presspassung besteht.

3. Kunstfuß nach einem der vorhergehenden Ansprüche,
**dadurch gekennzeichnet, dass**
das Federelement (50) im angeordneten Zustand im vorderen Bereich des Grundkörpers (60) und/oder im hinteren Bereich des Fersenkörpers (30) in Längsrichtung des Federelements (50) verschiebbar in einer Öffnung gelagert ist.

4. Kunstfuß nach einem der vorhergehenden Ansprüche,
**dadurch gekennzeichnet, dass**
der Grundkörper (60) einen Vorderfußkörper (70) mit dem vorderen Sohlenbereich (61) und einen Befestigungskörper (80), insbesondere zur Aufnahme eines Adapters (88), umfasst.

5. Kunstfuß nach einem der vorhergehenden Ansprüche,
insbesondere nach Anspruch 4,
**dadurch gekennzeichnet, dass**
der Vorderfußkörper (70) flexibel, insbesondere über einen Steg (65) mit dem Befestigungskörper (80) verbunden ist.

6. Kunstfuß nach einem der vorhergehenden Ansprüche,
**dadurch gekennzeichnet, dass**
der Fersenkörper (30) über einen Fersensteg (35) mit dem Grundkörper verbunden ist, wobei vorzugsweise der Fersensteg (35) derart ausgebildet ist, dass der Fersensteg (35) zumindest abschnittsweise das Federelement (50) umgreift.

7. Kunstfuß nach einem der vorhergehenden Ansprüche,
**dadurch gekennzeichnet, dass**
der vordere Sohlenbereich (61) zumindest abschnittsweise, vorzugsweise über mindestens 40% der Kontaktfläche, eine konvexe Form hat.

8. Kunstfuß nach einem der vorhergehenden Ansprüche,
**dadurch gekennzeichnet, dass**
der Grundkörper (60) ein Anschlagelement (62) umfasst, das sich nach unten, z.B. in einem Winkel von ca. 30 Grad erstreckt und auf dem das Federelemente (50) aufliegt.

9. Kunstfuß nach einem der vorhergehenden Ansprüche,
**dadurch gekennzeichnet, dass**
das Federelement (50) im angeordneten Zustand im Wesentlichen
parallel zur Adapterebene (AE) und/oder in einer nach vorne hin ansteigenden Ebene angeordnet ist.

10. Kunstfuß nach einem der vorhergehenden Ansprüche,
**dadurch gekennzeichnet, dass**
der Grundkörper (60) einen hinteren Anschlag (67) umfasst, der über einen Spalt, vorzugsweise von mindestens 1 cm, von dem Fersenkörper entfernt ist.

11. Kunstfuß nach einem der vorhergehenden Ansprüche, hergestellt in einem additiven Verfahren, insbesondere mittels 3D-Druck.

12. Kunstfuß nach einem der vorhergehenden Ansprüche,
**dadurch gekennzeichnet, dass**
der Grundkörper (60) und/oder der Fersenkörper (30) und/oder der/ein Vorderfußkörper (70) und/oder der/ein Befestigungskörper (80) und/oder einer der Stege und/oder das/ein Anschlagelement (62) und/oder die/eine Kappe (77) als ein integrales Element, insbesondere einstückig, ausgebildet sind.

13. Kunstfuß nach einem der vorhergehenden Ansprüche,
**dadurch gekennzeichnet, dass**
das Federelement (50) faserverstärkten Kunststoff umfasst und/oder aus GFK und/oder CFK hergestellt ist.

## Claims

1. Artificial foot, particularly a foot prosthesis (1), comprising a base body (60) having a front sole region (61) and a heel body (30) having a rear sole region (31),
**characterized in that**
the artificial foot comprises a slot (32) for receiving at least one retractable and/or insertable spring element (50), in particular at least one leaf spring,
and
in a disposed state, the spring element (50) engages in the base body (60) and the heel body (30) such that the heel body (30) is spring mounted relative to the base body (60).

2. Artificial foot according to Claim 1,
**characterized in that**
the spring element (50) in the disposed state is connected to the base body (60), in particular centrally, wherein there is preferably an adhesive connection and/or a screw connection and/or a latching connection and/or a connection via a press fit.

3. Artificial foot according to any one of the preceding claims,
**characterized in that**
the spring element (50) in the disposed state is mounted in an opening in the front region of the base body (60) and/or in the rear region of the heel body (30) so as to be movable in the longitudinal direction of the spring element (50).

4. Artificial foot according to any one of the preceding claims,
**characterized in that**
the base body (60) comprises a forefoot body (70) with the front sole region (61) and a mounting body (80), in particular for receiving an adapter (88).

5. Artificial foot according to any one of the preceding claims,
in particular according to Claim 4,
**characterized in that**
the forefoot body (70) is flexibly connected to the mounting body (80), in particular via a web (65).

6. Artificial foot according to any one of the preceding claims,
**characterized in that**
the heel body (30) is connected to the base body via a heel web (35), wherein the heel web (35) is preferably configured such that the heel web (35) surrounds the spring element (50) at least in sections.

7. Artificial foot according to any one of the preceding claims,
**characterized in that**
the front sole region (61) has a convex shape at least in sections, preferably over at least 40% of the contact surface.

8. Artificial foot according to any one of the preceding claims,
**characterized in that**
the base body (60) comprises a stop element (62) which extends downward, e.g. at an angle of approx. 30 degrees, and on which the spring element (50) rests.

9. Artificial foot according to any one of the preceding claims,
**characterized in that**
the spring element (50) in the disposed state is disposed substantially parallel to the adapter plane (AE) and/or in a plane which rises toward the front.

10. Artificial foot according to any one of the preceding claims,
**characterized in that**
the base body (60) comprises a rear stop (67), which is spaced apart from the heel body by a gap; preferably of at least 1 cm.

11. Artificial foot according to any one of the preceding claims, produced in an additive process, in particular by means of 3D printing.

12. Artificial foot according to any one of the preceding claims,
**characterized in that**
the base body (60) and/or the heel body (30) and/or the/a forefoot body (70) and/or the/a mounting body (80) and/or one of the webs and/or the/a stop element (62) and/or the/a cap (77) are formed as an integral element, in particular in one piece.

13. Artificial foot according to any one of the preceding claims,
**characterized in that**
the spring element (50) comprises fiber-reinforced plastic and/or is made of GFRP and/or CFRP.

## Revendications

1. Pied artificiel, en particulier prothèse de pied (1), comprenant un corps de base (60) avec une zone de semelle avant (61) et un corps de talon (30) avec une zone de semelle arrière (31),
**caractérisé en ce que**
le pied artificiel comprend un logement (32) destiné à recevoir au moins un élément élastique (50) pouvant être inséré et/ou enfiché, en particulier au moins un ressort à lame, et
l'élément élastique (50) s'engage, dans un état disposé, dans le corps de base (60) et le corps de talon (30), de sorte que le corps de talon (30) est monté élastiquement par rapport au corps de base (60).

2. Pied artificiel selon la revendication 1,
**caractérisé en ce que**
l'élément élastique (50) à l'état disposé est relié au corps de base (60), en particulier de manière centrale, de préférence par une liaison par collage et/ou par vissage et/ou par encliquetage et/ou par un ajustement serré.

3. Pied artificiel selon l'une des revendications précédentes,
**caractérisé en ce que**
l'élément élastique (50) à l'état disposé est monté dans une ouverture dans la zone avant du corps de base (60) et/ou dans la zone arrière du corps de talon (30) de manière à pouvoir coulisser dans la direction longitudinale de l'élément élastique (50).

4. Pied artificiel selon l'une des revendications précédentes,
**caractérisé en ce que**
le corps de base (60) comprend un corps d'avant-pied (70), avec la zone de semelle avant (61), et un corps de fixation (80), en particulier destiné à recevoir un adaptateur (88).

5. Pied artificiel selon l'une des revendications précédentes, en particulier selon la revendication 4,
**caractérisé en ce que**
le corps d'avant-pied (70) est relié de manière flexible, en particulier par une tige (65), au corps de fixation (80).

6. Pied artificiel selon l'une des revendications précédentes,
**caractérisé en ce que**
le corps de talon (30) est relié au corps de base par une tige de talon (35), la tige de talon (35) étant de préférence conçue de telle sorte que la tige de talon (35) entoure au moins partiellement l'élément élastique (50).

7. Pied artificiel selon l'une des revendications précédentes,
**caractérisé en ce que**
la zone de semelle avant (61) a une forme convexe au moins sur certaines parties, de préférence sur au moins 40 % de la surface de contact.

8. Pied artificiel selon l'une des revendications précédentes,
**caractérisé en ce que**
le corps de base (60) comprend un élément de butée (62) qui s'étend vers le bas, par exemple selon un angle d'environ 30 degrés, et sur lequel repose l'élément élastique (50).

9. Pied artificiel selon l'une des revendications précédentes,
**caractérisé en ce que**
l'élément élastique (50) à l'état disposé est disposé essentiellement parallèlement au plan de l'adaptateur (AE) et/ou dans un plan en pente ascendante vers l'avant.

10. Pied artificiel selon l'une des revendications précédentes,
**caractérisé en ce que**
le corps de base (60) comprend une butée arrière (67) qui est éloignée du corps de talon par une fente, de préférence d'au moins 1 cm.

11. Pied artificiel selon l'une des revendications précédentes, fabriqué par un procédé additif, en particulier par impression 3D.

12. Pied artificiel selon l'une des revendications précédentes,
**caractérisé en ce que**
le corps de base (60) et/ou le corps de talon (30) et/ou le/un corps d'avant-pied (70) et/ou le/un corps de fixation (80) et/ou l'une des tiges et/ou le/un élément de butée (62) et/ou le/un bout (77) sont réalisés sous la forme d'un élément intégré, en particulier d'une seule pièce.

13. Pied artificiel selon l'une des revendications précédentes,
**caractérisé en ce que**
l'élément élastique (50) comprend une matière plastique renforcée par des fibres et/ou est fabriqué en PRV et/ouPRFC.
